# FASCICULE DE BREVET EUROPEEN

(11) **EP 1 613 662 B1**
(45) Date de publication et mention de la délivrance du brevet: **15.01.2014**
(21) Numéro de dépôt: 03782560.1
(22) Date de dépôt: 14.11.2003
(51) Int. Cl.: C08B 37/08, A61K 35/56, A23L 1/00, A61P 19/02, A61P 27/02, A23J 3/34, A23L 1/307, A61K 38/01, C12N 1/00

(54) **PROCEDE D'OBTENTION D'ACIDE CHONDROITINE SULFURIQUE ET SES UTILISATIONS**
VERFAHREN ZUR HERSTELLUNG VON CHONDROITINSULFURSÄURE UND IHRE VERWENDUNGEN
NOVEL METHOD OF OBTAINING CHONDROITIN SULPHURIC ACID AND USES THEREOF

(30) Priorité: 15.11.2002 FR 0214348
(43) Date de publication de la demande: 11.01.2006
(73) Titulaire: Société La Biochimie Appliquée SA (Solabia), 93500 Pantin (FR)
(72) Inventeur: JOSSET, Gérard, F-92300 Levallois-Perret (FR)
(74) Mandataire: Célanie, Christian
(86) Numéro de dépôt international: PCT/FR2003/003375
(87) Numéro de publication internationale: WO 2004/046199

(56) Documents cités:
- EP-A- 0 274 946
- EP-A- 0 421 508
- EP-A- 0 549 048
- FR-A- 2 019 434
- FR-A- 2 756 828
- DATABASE WPI Section Ch, Week 199637 Derwent Publications Ltd., London, GB; Class B04, AN 1996-369700 XP002277895 & RU 1 609 153 C (VETERINARY PREPARATIONS RES CONTROL INST) 20 décembre 1995 (1995-12-20)
- DATABASE WPI Section Ch, Week 199227 Derwent Publications Ltd., London, GB; Class B04, AN 1992-223432 XP002277896 & JP 04 149137 A (NIPPON STEEL CHEM CO) 22 mai 1992 (1992-05-22)

## Description

La présente invention se rapporte au domaine de la chimie et plus particulièrement à celui de la chimie thérapeutique.

Elle concerne plus précisément un procédé de production et de purification de dérivés polyglucuroniques permettant d'aboutir dans des conditions avantageuses à des produits biologiques de haut degré de pureté.

Elle se rapporte également aux sous-produits formés au cours de ce procédé qui trouvent une utilisation dans la préparation de milieux de culture bactériologiques. Ces sous-produits permettent également d'obtenir des aminoacides qui trouvent une large utilisation dans l'alimentation, en diététique et pour des réactifs de laboratoire.

L'invention a spécifiquement pour objet un procédé de production et de purification d'acide chondroïtine-sulfurique à partir de tissus d'origine animale et principalement d'origine porcine ou bovine, qui consiste à soumettre des tissus cartilagineux et notamment des trachées-artère de ces animaux congelées, à un broyage puis à une hydrolyse enzymatique par une hydrolase spécifique puis à une filtration et ensuite à plusieurs dialyses contre de l'eau, séparation des acides aminés et des petits peptides et enfin précipitation par un alcanol pour obtenir l'acide chondroïtine-sulfurique purifié sous forme de sel de sodium.

Les phases les plus importantes du procédé selon l'invention, se situent donc d'une part dans l'hydrolyse enzymatique, et d'autre part dans la succession d'opérations de dialyse. L'hydrolyse enzymatique est effectuée à l'aide d'une protéase alcaline du type serine obtenue à partir de cultures de Bacillus licheniformis et plus particulièrement à partir d'une souche sélectionnée de Bacillus licheniformis commercialisée sous la dénomination PROLYVE 1000 par la société LYVEN.

La protéase produite à partir de cette souche est active et stable en solution sur une vaste zone de pH s'étendant de 8,0 à 11,0. Toutefois son activité optimale se situe entre 9,0 et 10,5. Sa température optimale d'activité se situe entre 55° C et 60° C suivant la durée de l'hydrolyse. L'enzyme est totalement inactivée après 10 à 15 minutes de chauffage à 90° C.

A côté de son activité endopeptidasique cet enzyme de Bacillus licheniformis présente une action estérasique prononcée qui permet l'hydrolyse de substrats synthétiques.

Les agents chelatants sont sans effet sur l'enzyme de Bacillus licheniformis qui est par ailleurs, inhibé par des agents d'acylation comme le fluorure de phénylméthane sulfonyle et le disipropyl phosphofluoridate.

L'enzyme de Bacillus licheniformis n'est pas inhibé par l'inhibiteur trypsique de soja mais il est sensible à l'inhibiteur de la pomme de terre et à l'inhibiteur de certaines céréales.

L'enzyme de Bacillus licheniformis manifeste une forte activité endopeptidasique. Il a déjà de larges applications dans le domaine de l'hydrolyse des protéines animales ou végétales. Son activité conduit surtout à la formation de petits peptides solubles dans l'eau. Au contraire, il ne produit que de faibles concentrations en acides aminés libres.

L'optimum d'action se situe comme indiqué vers 10 suivant le type de protéine à hydrolyser. Il a été nécessaire cependant dans le cas précis à considérer, d'ajuster le pH pour trouver l'optimum d'action pour le substrat considéré. Dans le cas de cartilages de trachée artère selon l'invention, cet optimum de pH se situe entre 8 et 9 et de préférence à 8,5. La température d'hydrolyse enzymatique se situe vers 60° C pour parfaire l'hydrolyse des protéines tout en évitant que l'acide chondroïtine sulfurique ne soit attaqué.

Après hydrolyse enzymatique, on procède à une thermolyse de l'enzyme à pH 6,0 et à 97° C pendant 20 à 60 minutes, de préférence pendant 30 minutes. Du fait de conditions physiques modérées, l'acide chondroïtine sulfurique ne subit pas de dégradation.

La dialyse ou l'ultrafiltration est précédée d'une étape de filtration. Elle conduit à l'élimination des acides aminés et des oligo-peptides formés lors de l'hydrolyse des protéines, qui passent dans l'ultrafiltrat.

L'acide chondroïtine sulfurique ainsi obtenu est isolé par précipitation en milieu acide (pH 3,0) et éthanolique à l'aide d'un éthanol de titre éthanolique de l'ordre de 96 % (w/v).

Le produit est finalement séché sur un appareil de séchage industriel. Le procédé est parfaitement reproductible, quelles que soient les variations dans la qualité des matériaux de départ. Le procédé a été essayé sur 17 lots, 14 lots d'origine bovine et 3 lots d'origine porcine et a conduit à des lots d'acide chondroïtine-sulfurique de très haute pureté se situant en règle générale au dessus de 97 % en acide chondroïtine-sulfurique en sec pour cent grammes.

L'acide chondroïtine-sulfurique, sel de sodium (ACSNa) est un polymère dont les monomères, en proportions non définies, sont les sels disodique des isomères 4 ou 6 de l'acide chondroïtine sulfurique, disaccharide constituée d'acide β glucuronique et de N-acétylgalactosamine (liaison β 1 → 3). L'ACSNa est donc constitué des esters sulfuriques en 4 ou 6 de la N-acétylgalactosamine du monomère, il est dihydraté et répond à la formule C₁₄H₁₉O₁₄N Na₂S, 2H₂O.

La structure du monomère se définit comme suit :

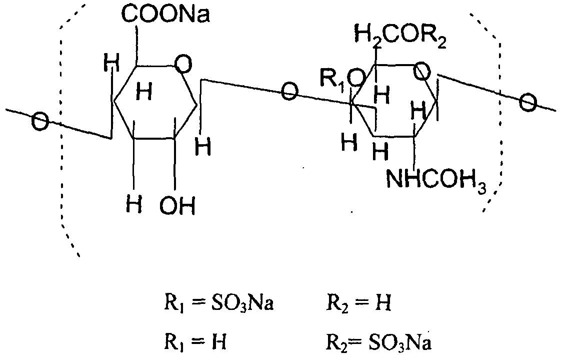

Le produit est un mélange de deux polysaccharides dont les monomères sont : l'acide β-D GLUCURONIQUE et la N-ACETYL GALACTOSAMINE 4-SULFATE liés en β (1→3)

Dans les tissus, les polymères de l'acide chondroïtine-sulfurique sont liés d'une manière covalente à des chaînes protéiques pour former des protréoglycanes. La littérature enseigne déjà que l'acide chondroïtine sulfurique peut être obtenu à partir de matières premières d'origines variées comme des cartilages d'animaux tel que les bovins, les ovins, les porcins, les chevaux, les requins, les poissons et même les baleines ou les écailles de poissons.

Une des sources principales d'acide chondroïtine est l'extraction d'organes cartilagineux d'origine bovine ou porcine et notamment de trachées d'origine bovine et porcine.

Cependant des développements récents, relatifs en particulier à des contaminations par des virus ou des prions, ont soulevé des problèmes d'innocuité et ont conduit à envisager d'autres sources de matière premières, d'origine aviaire, c'est-à-dire exempts de toute maladie propre aux bovins.

Cette considération largement décrite dans le brevet français 2.756.828 s'est sensiblement estompée et ne présente plus le même problème d'acuité.

Le procédé selon l'invention vise par une hydrolyse enzymatique spécifique à débarrasser l'acide chondroïtine sulfurique des glycoprotéines auxquelles il est associé à l'état naturel. L'enzyme responsable de cette hydrolyse doit présenter une activité spécifique empêchant l'attaque de la molécule d'acide chondroïtine sulfurique ou de ses polymères, de manière à éviter le fractionnement irréversible de la molécule.

La littérature fournit déjà un certain nombre de références relatives à la production de l'acide chondroïtine sulfurique à partir des différentes matières premières animales. C'est ainsi qu'un certain nombre de brevets décrit un procédé d'hydrolyse enzymatique utilisant des protéases (CTPP brevet français 2.804.022, Hokkaïdo JP 2000/273102, Pierre Fabre BF2.756.828, Biomed Research USP 4.302.577, Agency of Ind-Sci and Technology USP 6.342.367).

La littérature décrit également des procédures d'ultrafiltration utilisant des membranes variées (Hokkaïdo JP2000/273102, Pierre Fabre 2.736.828) ou la dialyse (CTPP BF2.804.022, Sanrit Sutsu KK JP73/27632).

Cette multiplication de techniques de production et surtout la description du brevet français 2.756.828 indique clairement qu'aucun des procédés actuellement décrits n'est pleinement satisfaisant ou conduit à des composés de qualité régulière ou satisfaisante. Selon le dernier brevet 2.756.828 l'acide chondroïtine sulfurique obtenu se définit par un poids moléculaire compris entre 20 et 21.000 daltons. Les données de la littérature renseignent habituellement sur une valeur de 8.000 à 50.000.

Les essais ont montré qu'il était difficile d'obtenir un acide chondroïtine sulfurique de bonne qualité. Le procédé selon l'invention permet justement de résoudre ce problème.

Le produit obtenu selon le procédé de la présente invention se caractérise par un poids moléculaire moyen s'échelonnant de 15.000 à 30.000 daltons.

Les méthodes de dosage effectuées en routine permettent de définir les critères de pureté :
- la description de l'aspect du produit, sa solubilité dans l'eau et la taille maximale des particules (97 % < 500µm),
- son identification par caractérisation de l'anion acide chondroïtine-sulfurique (spectre IR et réaction au chlorure de cétylpyridinium) et du cation sodium (réaction b de la Pharmacopée Européenne 2.3.1.),
- la vérification d'une salification suffisante par mesure du pH (en solution aqueuse à 1 %),
- la détermination de la perte à la dessication qui couvre les deux molécules d'eau du monomère et l'hygroscopicité du polymère, caractéristique qui explique l'amplitude des spécifications -10 à 13 %- alors que l'eau d'hydratation ne représente qu'environ 6,3 %. La détermination de cette perte à la dessication est indispensable pour que les résultats des déterminations quantitatives soient rapportés au produit desséché pour les rendre significatifs,
- des teneurs limites en impuretés : métaux lourds, sulfates libres, chlorures,
- des dosages liés à la formule moléculaire (résultats rapportés au produit desséché),
- dosage du soufre total qui couvre essentiellement l'atome de soufre de l'ACSNa mais aussi celui des sulfates libres,
- dosage de l'azote total, qui couvre essentiellement l'atome d'azote de l'ACSNa, mais aussi celui des impuretés protéiques,
- détermination des cendres sulfuriques qui représentent essentiellement les deux atomes de sodium de l'ACSNa, mais aussi des impuretés métalliques,
- la détermination de la teneur en ACSNa anhydre, rapportée au produit desséché.

Un autre objet de l'invention réside dans l'obtention de peptones au cours de l'hydrolyse enzymatique. L'intérêt présenté par l'hydrolyse enzymatique réside dans le fait que les diverses protéines et en particulier les glycoprotéines sont dégradées en petites molécules telles que les peptones et même en amino acides.

Les peptones sont des produits solides ou granuleux, de couleur blanc jaunâtre, de saveur légèrement amère. Ils sont solubles dans l'eau, insolubles dans l'alcool concentré. Les peptones sont constituées par des polypeptides de faible poids moléculaire et par des amino acides.
Il peut être important de séparer les amino acides notamment par passage sur une résine échangeuse d'ions et élution par une solution ammoniacale.

Les peptones trouvent un emploi comme source d'azote et élément nutritif pour les bouillons de culture, notamment la peptone gélosée.

Les amino acides que l'on peut séparer consistent essentiellement en de l'argine, de la lysine et de la tyrosine. Après purification ils constituent des matières premières précieuses en diététique, en pharmacie ou en cosmétique.

L'acide chondroïtine sulfurique trouve une utilisation importante en thérapeutique dans le traitement de l'arthrose et dans le traitement des affections oculaires.

Le procédé selon l'invention se définit comme suit à la lumière des exemples.

### Etape 1

### Broyage des trachées congelées

Les trachées utilisées sont collectées auprès d'abattoirs agréés. On procède à une vérification visuelle des trachées congelées puis on procède à un broyage dans un hachoir à couteaux. On utilise 3.000 kg par lot.

### Etape 2

### Hydrolyse enzymatique

On opère en cuve thermostatée à 60° C en introduisant les 3.000 kg de broyat de trachées et de l'eau (4.500 litres) puis une solution de protéase alcaline (Prolyve 1000) titrant plus de 3.000 U/mg. On ajuste le pH à 8,5 par addition d'une solution d'hydroxyde de sodium 10 M. On laisse l'hydrolyse s'effectuer pendant 5 heures, à cette température.

### Etape 3

### Thermolyse de l'enzyme

On amène le pH du jus d'hydrolyse maintenu à 60° C, en ajoutant de l'acide chlorhydrique 12 M puis on chauffe 30 minutes à 97° C. Le chauffage produit en même temps que l'hydrolyse, une décontamination du milieu.

### Etape 4

### Filtration et ultra filtration

On laisse refroidir les jus d'hydrolyse thermolysés et on procède à une décantation, puis à une filtration sur filtre presse.

On procède sur les jus filtrés à une ultra filtration sur membrane PW120 PO ayant un seuil de coupure d'environ 20.000 daltons. Cette opération permet de concentrer la solution en éliminant les acides aminés et les petits peptides. On peut effectuer dans ce cas au préalable, un traitement alcalin pour les trachées d'origine bovine. On maintient 1 heure entre 20 et 25° C.

Le produit est envoyé par l'intermédiaire d'une canalisation dédiée jusqu'à la cuve de lancement. Le jus d'hydrolyse titre 12 % environ d'extrait sec. La température est de 60° C, le pH est de 9. On utilise un volume (V1) de 5.000 litres.

### Concentration

Cette étape permet de concentrer le produit tout en éliminant les éléments non souhaités tels que les protéines, les sels minéraux...
débit de l'ultra filtration environ 1.000-1.500 litres par heure
le pH est maintenu à 9 et la température à 60° C
Le volume final d'élution (V1) est de 3.000 litres.

### 1 ère dialyse

### (Dialyse en automatique - dialyse grossière)

On envoie de l'eau purifiée à 60° C en continu, tout en procédant à l'ultra filtration. Le volume de la cuve de lancement demeure constant.
Volume total d'eau ajoutée : 3.600 litres.
débit de l'ultra filtration : environ 1.500 litres par heure
pH 9
Volume final (V1) : 2.200 litres

### 2ème dialyse

On effectue la dialyse sur la totalité des solutions aqueuses. Il s'agit donc d'une dialyse fine. On envoie de l'eau purifiée à 60° C en une seule fois pour favoriser une dilution importante, de façon à réaliser une viscosité plus faible et une meilleure dilution des électrolytes. Cette étape permet d'éliminer plus facilement les protéines.
Volume total d'eau ajoutée : 3.600 litres.
La température du milieu est de 50° C.
Le débit de l'ultra filtration est de 1.500 à 2.000 litres par heure.
La valeur du pH est de l'ordre de 9.
Le volume final est de 2.200 litres

### 3ème dialyse

On effectue la dialyse sur la totalité de l'éluat. On procède comme pour la 2ème dialyse. Volume total d'eau ajoutée : 2.000 litres.
La température est de 50° C.
Le débit de l'ultra filtration est de 1.500 à 2.000 litres par heure. La valeur du pH est de 9.
Le volume final est de 2.000 litres.
Le débit du produit d'ultra filtration est de 1.500 à 2.000 litres par heure. Le pH du milieu est de 9.
Le volume final est de 2.200 litres.
On procède ensuite à une précipitation par l'éthanol. On ajoute de l'acide chlorhydrique 12 M jusqu'à pH 3,0 puis du chlorure de sodium jusqu'à une concentration finale de 80 grammes par litre. Après filtration, on recueille une solution limpide dans une cuve de précipitation, on ajoute de l'éthanol à raison de 1,3 fois le volume de filtrat. On soutire le surnageant hydro-alcoolique et le précipité d'acide chondroïtine sulfurique est transféré dans une autre cuve. On décante et élimine le surnageant puis on recueille le précipité qu'on purifie par 3 lavages successifs à l'aide d'éthanol dilué, de concentrations croissantes progressives (70°, 75° et 80°). Le produit est ensuite essoré et rincé 2 fois à l'éthanol.
Le produit est ensuite séché dans un dessicateur équipé d'une pompe à vide et d'une régulation thermique jusqu'à obtention d'une perte à la dessication comprise entre 10 et 13 %.
Le séchage est effectué à 60° C sous vide.
L'acide chondroïtine sulfurique obtenu dans ces conditions se présente sous forme d'une poudre blanche à blanc-cassé, soluble dans l'eau.
La taille des particules obtenue par tamisage montre qu'il y a 97 % des particules <500 µm.

Le pH en solution aqueuse à 1 % est de 5,5 à 7,5. Les données spectrales sont conformes à celles d'un témoin (spectre IR en pastilles de BrK comparable à celui d'un témoin, bandes caractéristiques à 930-920 cm⁻¹, 860-850cm⁻¹ et 730-710cm⁻¹ pour l'acide chondroïtine 4-sulfurique et à 1.660-1.600cm⁻¹ et 1.250-1270-cm⁻¹ pour l'acide chondroïtine 6-sulfurique).

La teneur en soufre varie de 5,0 à 7,0 % rapporté au produit desséché.
L'azote total varie de 2,5 à 4,5 % rapporte au produit desséché.
Le taux de protéines est inférieur à 2 % déterminée par la méthode de Lowry.
Les cendres sulfuriques varient de 20 à 30 %.
Le dosage titrimétrique de l'acide chondroïtine sulfurique indique une teneur de 95 à 102 % rapporté au produit desséché.

La détermination de la contamination bactérienne indique un nombre de germes aérobies viables totaux inférieur à 1.000 CFU par gramme.

Le tableau I suivant récapitule les normes obtenues sur 17 lots différents montrant la parfaite répétitivité des essais.

**TABLEAU**

| Lot N° | Date Product. | Taille du Lot en Kilogr. | Origine des Trachées | Perte Dessiccat p. cent | pH Sol. 1% | Identific. ABC | Soufre / sec | Azote Total /sec | Protéines | Cendres Sul./ sec | Chlorures | Dosage ACS / sec |
|---|---|---|---|---|---|---|---|---|---|---|---|---|
| | | | | | | +++ | p. cent 5,0 â 7,0 | p. cent 2,5 à 4,5 | p. cent ≤ 2,0 | p.cent (20 á 30) | p. cent ≤ 1,0 | p. cent 95 à 102 |
| 8A746 | 01/98 | 534,8 | Bovine | 11 | 5,5 | +++ | 5,4 | 2,9 | 0,9 | 26 | 0,5 | 97 |
| 8B916 | 02/98 | 1000,0 | Bovine | 10 | 6,0 | +++ | 5,1 | 2,9 | 0,8 | 27 | 0,5 | 96 |
| 8B999 | 02/98 | 1010,6 | Bovine | 10 | 5,2 | +++ | 5,7 | 2,7 | 0,8 | 26 | 0,6 | 97 |
| 8C201 | 03/98 | 1007,0 | Bovine | 11 | 6,1 | +++ | 5,6 | 3,3 | 1,1 | 27 | 0,5 | 98 |
| 8C359 | 03/98 | 1010,5 | Bovine | 12 | 6,5 | +++ | 5,2 | 2,8 | 1,4 | 27 | 0,3 | 96 |
| 8D569 | 04/98 | 1018,9 | Bovine | 12 | 7,3 | +++ | 5,2 | 3,1 | 1,3 | 28 | 0,4 | 97 |
| 8E760 | 05/98 | 1033,5 | Bovine | 10 | 7,1 | +++ | 5,2 | 3,0 | 1,3 | 28 | 0,5 | 99 |
| SE833 | 06/98 | 400,0 | Porcine | 10 | 7,0 | +++ | 5,0 | 3,1 | 1,7 | 26 | 0,7 | 98 |
| 8F926 | 06/98 | 1011,3 | Bovine | 11 | 6,1 | +++ | 5,1 | 3,1 | 0,9 | 27 | 0,5 | 98 |
| 8G066 | 07/98 | 1005,7 | Bovine | 12 | 6,0 | +++ | 5,0 | 3,0 | 1,9 | 27 | 0,5 | 98 |
| 8G098 | 07/98 | 1013,5 | Porcine | 11 | 5,2 | +++ | 5,0 | 3,2 | 1,4 | 26 | 0,6 | 98 |
| 8G166 | 07/98 | 1029,5 | Bovine | 10 | 5,6 | +++ | 5,5 | 3,2 | 2,3 | 25 | 0,4 | 97 |
| 8H300 | 08/98 | 1021,1 | Bovine | 11 | 6,1 | +++ | 5,1 | 3,2 | 1,4 | 26 | 0,2 | 100 |
| 8J425 | 09/98 | 1001,1 | Porcine | 11 | 7,0 | +++ | 5,1 | 3,3 | 1,6 | 24 | 0,3 | 99 |
| 8J454 | 09/98 | 1018,2 | Bovine | 11 | 6,0 | +++ | 5,4 | 3,2 | 1,7 | 25 | 0,2 | 98 |
| 8J578 | 10/98 | 1014,1 | Bovine | 10 | 6,7 | +++ | 5,0 | 3,2 | 1,4 | 26 | 0,3 | 98 |
| 8K650 | 10/98 | 1012,6 | Bovine | 10 | 6,2 | +++ | 5,1 | 3,2 | 1,4 | 26 | 0,3 | 99 |

## Revendications

1. Procédé de production et de purification de l'acide chondroïtine sulfurique dans lequel on soumet des tissus cartilagineux d'origine animale à une hydrolyse enzymatique puis après neutralisation, à une ultrafiltration et à une dialyse et dans lequel on précipite l'acide chondroïtine sulfurique par addition d'un alcanol soluble dans l'eau, **caractérisé en ce qu'**on effectue l'hydrolyse enzymatique des tissus cartilagineux d'origine bovine ou porcine au moyen d'une protéase alcaline extraite de Bacillus licheniformis puis la thermolyse de l'enzyme à chaud en milieu acide, la concentration de l'acide chondroïtine sulfurique par ultrafiltration et dialyses répétées et la précipitation de l'acide chondroïtine sulfurique par addition d'un volume déterminé d'éthanol, et **en ce que** l'acide chondroïtine sulfurique obtenu présente un poids moléculaire moyen s'échelonnant de 15000 à 30000 Daltons.

2. Procédé selon la revendication 1, **caractérisé en ce que** la protéase extraite de Bacillus licheniformis est le produit commercialisé par la société Lyven.

3. Procédé selon la revendication 1 ou la revendication 2, **caractérisé en ce que** pour le substrat considéré l'optimum de l'action enzymatique se situe à un pH compris entre 8 et 9.

4. Procédé selon l'une des revendications précédentes **caractérisé en ce que** l'optimum d'action se situe à un pH de 8,5.

5. Procédé selon l'une des revendications précédentes **caractérisé en ce que** l'hydrolyse enzymatique est effectuée à une température voisine de 60°C.

6. Procédé selon l'une des revendications précédentes dans lequel on effectue une thermolyse de l'enzyme à pH 6,0 et à 97°C.

7. Procédé selon la revendication 6 dans lequel la thermolyse est effectuée pendant 20 à 60 minutes.

8. Procédé selon la revendication 1, dans lequel on procède à trois dialyses successives pour concentrer le milieu en acide chondroïtine sulfurique et obtenir un produit présentant une pureté supérieure à 95%.

9. Procédé selon la revendication 1, dans lequel on précipite l'acide chondroïtine sulfurique par addition d'un volume d'éthanol en quantité égale à 1,3 fois le volume de solution filtrée.

10. Procédé selon la revendication 1, dans lequel après purification et séchage on obtient un acide chondroïtine sulfurique de qualité pharmaceutique.

11. Procédé selon la revendication 1, dans lequel on sépare la fraction contenant des petits peptides constitués par des peptones et des acides aminés, après dialyse.

## Patentansprüche

1. Verfahren zur Herstellung und Reinigung von Chondroitin-Schwefelsäure, bei welchem vom Tier stammende knorpelige Gewebe einer enzymatischen Hydrolyse und dann, nach Neutralisation, einer Ultrafiltration und einer Dialyse unterzogen werden, und bei welchem die Chondroitin-Schwefelsäure durch Zugabe von in Wasser löslichem Alkanol gefällt wird, **dadurch gekennzeichnet, dass** die enzymatische Hydrolyse der vom Rind oder Schwein stammenden knorpeligen Gewebe mittels einer alkalischen Protease, die aus Bacillus licheniformis extrahiert ist, und dann die Thermolyse des Enzyms unter Wärme in saurer Umgebung, die Anreicherung von Chondroitin-Schwefelsäure durch Ultrafiltration und wiederholte Dialysen und die Fällung von Chondroitin-Schwefelsäure durch Zugabe eines vorgegebenen Volumens von Ethanol durchgeführt werden, und dass die erzielte Chondroitin-Schwefelsäure ein mittleres Molekulargewicht aufweist, das sich von 15000 bis 30000 Dalton staffelt.

2. Verfahren nach Anspruch 1, **dadurch gekennzeichnet, dass** die Protease, die aus Bacillus licheniformis extrahiert ist, das Produkt ist, welches von der Firma Lyven vertrieben wird.

3. Verfahren nach Anspruch 1 oder Anspruch 2, **dadurch gekennzeichnet, dass** für das betreffende Substrat das Optimum der enzymatischen Aktion sich bei einem pH von zwischen 8 und 9 befindet.

4. Verfahren nach einem der vorhergehenden Ansprüche, **dadurch gekennzeichnet, dass** das Aktionsoptimum sich bei einem pH von 8,5 befindet.

5. Verfahren nach einem der vorhergehenden Ansprüche, **dadurch gekennzeichnet, dass** die enzymatische Hydrolyse bei einer Temperatur von 60°C ausgeführt wird.

6. Verfahren nach einem der vorhergehenden Ansprüche, bei dem eine Thermolyse des Enzyms bei pH 6,0 und bei 97°C ausgeführt wird.

7. Verfahren nach Anspruch 6, bei dem die Thermolyse während 20 bis 60 Minuten ausgeführt wird.

8. Verfahren nach Anspruch 1, bei dem drei Dialysen nacheinander vorgenommen werden, um das Milieu mit Chondroitin-Schwefelsäure anzureichern und ein Produkt zu erzielen, welches eine Reinheit von über 95% aufweist.

9. Verfahren nach Anspruch 1, bei dem die Chondroitin-Schwefelsäure durch Zugabe eines Volumens von Ethanol gefällt wird, welches in der Menge dem 1,3 fachen Volumen der filtrierten Lösung entspricht.

10. Verfahren nach Anspruch 1, bei dem nach Reinigung und Trocknung eine Chondroitin-Schwefelsäure von pharmazeutischer Qualität erzielt wird.

11. Verfahren nach Anspruch 1, bei dem die Fraktion, welche kleine Peptide enthält, die sich aus Peptonen und Aminosäuren zusammensetzen, nach der Dialyse separiert wird.

## Claims

1. Process to produce and purify chondroitin sulphuric acid in which cartilaginous animal tissues are subjected to an enzymatic hydrolysis, then after being neutralised, to an ultrafiltration and a dialysis and in which the chondroitin sulphuric acid is precipitated by the addition of a watersoluble alkanol, **characterised in that** the enzymatic hydrolysis of cartilaginous tissues of bovine or porcine origin is performed by means of an alkaline protease derived from Bacillus licheniformis, the thermolysis of the enzyme is thereafter performed in a hot acid medium, the chondroitin sulphuric acid is concentrated by means of repeated ultrafiltration and dialysis and precipitated by the addition of a pre-determined volume of ethanol, and **in that** the chondroitin sulphuric acid obtained has a mean molecular weight ranging from 15,000 to 30,000 Daltons.

2. A process according to Claim 1, **characterised in that** the protease derived from the Bacillus licheniformis is the product sold by the company Lyven.

3. A process according to Claims 1 or 2, **characterised in that** for the substrate considered the optimum of enzyme action is at a pH of between 8 and 9.

4. A process according to one of the above Claims, **characterised in that** the optimum of action is at a pH of 8.5.

5. A process according to one of the above Claims, **characterised in that** the enzymatic hydrolysis is performed at a temperature close to 60°C.

6. A process according to one of the above Claims, **characterised in that** a thermolysis of the enzyme is performed at a pH of 6.0 and a temperature of 97°C.

7. A process according to Claim 6, **characterised in that** the thermolysis is performed for 20 to 60 minutes.

8. A process according to Claim 1, **characterised in that** three successive dialyses are performed in order to concentrate the medium in chondroitin sulphuric acid and obtain a product with a purity of over 95%.

9. A process according to Claim 1, **characterised in that** the chondroitin sulphuric acid is precipitated by the additional of a volume of ethanol equal to 1.3 times the volume of the filtered solution.

10. A process according to Claim 1, **characterised in that** after purification and drying a pharmaceutical grade chondroitin sulphuric acid is obtained.

11. A process according to Claim 1, **characterised in that** the fraction containing small peptides constituted by peptones and amino acids is separated after dialysis.
